(19) Europäisches Patentamt
European Patent Office
Office européen des brevets

(11) Veröffentlichungsnummer: **0 122 479**
**A1**

(12) # EUROPÄISCHE PATENTANMELDUNG

(21) Anmeldenummer: **84102923.4**

(22) Anmeldetag: **16.03.84**

(51) Int. Cl.³: **C 07 C 49/175**
C 07 C 45/00

(30) Priorität: **18.03.83 DE 3309811**

(43) Veröffentlichungstag der Anmeldung:
**24.10.84 Patentblatt 84/43**

(84) Benannte Vertragsstaaten:
**AT BE CH DE FR GB IT LI NL**

(71) Anmelder: **WACKER-CHEMIE GMBH**
**Prinzregentenstrasse 22**
**D-8000 München 22(DE)**

(72) Erfinder: **Petersen, Hermann, Dr. Dipl.-Chem.**
**Paganiniweg 14**
**D-8263 Burghausen(DE)**

(72) Erfinder: **Deinhammer, Wolfgang, Dr. Dipl.-Ing.**
**Röntgenstrasse 32**
**D-8263 Burghausen(DE)**

(54) **3-Chlor-1,1-dialkoxyacetone und Verfahren zu ihrer Herstellung.**

(57) Gegenstand der Erfindung sind 3–Chlor–1, 1–dialkoxy–acetone der Formel

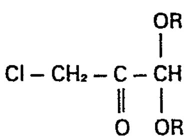

worin R Alkylreste mit 1 bis 5 C-Atomen bedeutet, die aufgrund ihrer funktionellen Gruppen wertvolle Zwischenprodukte insbesondere für die Synthese von Folsäurederivaten darstellen. Diese Verbindungen können durch Umsetzung von Gemischen aus 3–Chlor– 1–Hydroxyimino–aceton und einem Alkanol mit Alkylnitriten in Gegenwart eines anorganischen Elektronenpaarakzeptors, wie Chlorwasserstoff bei Temperaturen im Bereich von −25°C bis +60°C und Reinigung der so erhaltenen Rohprodukte durch Destillation unter vermindertem Druck hergestellt werden. Als Reaktionspartner können hierbei 3–Chlor– 1–hydroxyimino–aceton, das unmittelbar vor Gebrauch durch Umsetzung von 4–Chloracetessigsäure mit Natriumnitrit in wässeriger Lösung und anschließender Extraktion mit einem organischen Lösungsmittel erhalten worden ist, sowie Alkanole und Alkylnitrite, die jeweils identische Alkylreste mit 1 bis 5 C–Atomen enthalten, verwendet werden. Die so herstellbaren Verbindungen sind bei Raumtemperatur stabil und längere Zeit ohne Zersetzungsneigung lagerbeständig.

EP 0 122 479 A1

Croydon Printing Company Ltd.

0122479

W A C K E R - C H E M I E
G M B H

München, den 2. März 1983
Dr.Wg/rei

Wa 8245-C

3-Chlor-1,1-dialkoxyacetone und Verfahren zu ihrer Herstellung

Gegenstand der Erfindung sind 3-Chlor-1,1-dialkoxyacetone der Formel

$$Cl - CH_2 - \underset{\underset{O}{\|}}{C} - \overset{\overset{OR}{|}}{\underset{\underset{OR}{|}}{CH}}$$

worin R Alkylreste mit 1 bis 5 C-Atomen bedeutet und Verfahren zu ihrer Herstellung.

Die Herstellung von 1,1-Dialkoxy-acetonen (Pyruvaldehyd-1-dialkylacetalen oder Methylglyoxal-1-dialkylacetalen) durch Umsetzung von Aceton und Alkanolen mit Nitrosierungsmitteln in Gegenwart von sauren Katalysatoren ist bekannt (vgl. DE-PS 12 52 193), wobei es sich im Falle des 1,1-Dimethoxy-acetons als zweckmäßig erwiesen hat, dieses Verfahren in 2 Stufen, das heißt unter Isolierung des als Zwischenprodukt anfallenden Isonitrosoacetons (Hydroxyimino-aceton) durchzuführen (vgl. US-PS 4.158.019).

Es ist ferner bekannt, daß aus 1,1-Diethoxy-aceton durch Einwirkung von Brom in Schwefelkohlenstofflösung in Gegenwart von wasserfreiem Natriumcarbonat 3-Brom-1,1-diethoxy-aceton entstehen soll, das aber nicht näher beschrieben wird (vgl. US-PS 2.436.073).

Die Umsetzung von Chloracetonen anstelle von Aceton mit Nitrosierungsmitteln in Gegenwart von sauren Katalysatoren führt praktisch ausschließlich zur Bildung von 1-Chlor-1-

hydroxyimino-aceton (Brenztraubensäurechlorid-1-oxim oder Pyruvohydroximoylchlorid) der Formel

$$CH_3 - \underset{\underset{O}{\|}}{C} - \underset{\underset{Cl}{|}}{C} = NOH$$

(vgl. US-PS 2.844.630 und DE-OS 21 11 459).

Die Herstellung des isomeren 3-Chlor-1-hydroxyimino-acetons ist indessen durch Chlorierung von Diketen in Tetrachlorkohlenstofflösung bei Temperaturen $< 0^oC$ unter Bildung von Chloracetoacetylchlorid als Zwischenprodukt und anschließender Behandlung mit wäßriger Natriumnitritlösung bei Temperaturen $< 5^oC$ möglich. Diese Verbindung ist sehr unbeständig und auch bei $-20^oC$ nur begrenzt lagerfähig, da sie sich unter Bildung von Blausäure zersetzt (vgl. E.C. Taylor et al in J. Org. Chem. Vol. 38 (1973), Seite 806 ff).

Eigenschaften und Herstellung von 3-Chlor-1,1-dialkoxy-acetonen, die aufgrund ihrer funktionellen Gruppen wertvolle Zwischenprodukte insbesondere für die Synthese von Folsäurederivaten darstellen, sind demnach nicht bekannt.

3-Chlor-1,1-dialkoxy-acetone werden vorteilhaft dadurch hergestellt, daß Gemische aus 3-Chlor-1-hydroxyimino-aceton und einem Alkanol mit Alkylnitriten in Gegenwart eines anorganischen Elektronenpaarakzeptors bei Temperaturen im Bereich von $-25^oC$ bis $+60^oC$ umgesetzt und die so erhaltenen Rohprodukte durch Destilllation unter vermindertem Druck gereinigt werden.

Das als Ausgangsmaterial verwendete 3-Chlor-1-hydroxyimino-aceton wird vorteilhaft unmittelbar vor Gebrauch hergestellt, wobei sich das folgende Verfahren besonders bewährt hat:
Eine Lösung von handelsüblicher 4-Chloracetessigsäure in verdünnter Salzsäure wird bei Temperaturen von $0^o$ bis $+5^oC$ in

eine wäßrige Lösung von Natriumnitrit eingetragen und das Reaktionsgemisch anschließend mit einem organischen Lösungsmittel, wie Diisopropylether extrahiert. Nach Waschen und Trocknen der organischen Phase und Entfernen des Lösungsmittels unter vermindertem Druck wird 3-Chlor-1-hydroxyimino-aceton in kristalliner Form in Ausbeuten von $>$ 80 %, bezogen auf eingesetzte 4-Chlor-acetessigsäure erhalten, das ohne weitere Reinigung für das erfindungsgemäße Verfahren eingesetzt werden kann.

Als Reaktionspartner für das erfindungsgemäße Verfahren sind je Mol des 3-Chlor-1-hydroxyimino-acetons mindestens 1 Mol des Alkanols und mindestens 1 Mol des Alkylnitrits erforderlich, wobei zweckmäßig die 1 bis 5 C-Atome enthaltenden Alkylreste von Alkanol und Alkylnitrit jeweils identisch sind um die Bildung von gemischten Dialkoxy-acetonen zu vermeiden. Vorteilhaft werden jedoch mindestens 5 bis 20 Mol des Alkanols je Mol des 3-Chlor-1-hydroxyimino-acetons eingesetzt, wobei das Alkanol gleichzeitig als Lösungsmittel dient. Von den entsprechenden Alkylnitriten werden vorteilhaft jeweils 1,0 bis 1,2 Mol je Mol 3-Chlor-1-hydroxyimino-aceton verwendet. Der Einsatz größerer Mengen an Alkylnitriten bringt praktisch keinen Vorteil, da hierbei bereits die Chlormethylgruppe angegriffen wird. Außerdem ist die Gegenwart eines Elektronenpaarakzeptors erforderlich, worunter starke anorganische Säuren und Lewis-Säuren zu verstehen sind, wobei sich Chlorwasserstoff besonders bewährt hat, der vorteilhaft in Mengen von 0,01 bis 2 Mol je Mol des 3-Chlor-1-hydroxyimino-acetons eingesetzt wird.

Die erforderlichen Reaktionstemperaturen sind innerhalb des definierten Bereichs abhängig von der Art der Alkylreste des verwendeten Alkanols und des Alkylnitrits. So haben sich beispielsweise für die Herstellung von 3-Chlor-1,1-dimethoxyaceton unter Einsatz von Methanol und Methylnitrit Temperaturen von $-15^{o}C$ bis $0^{o}C$ und abschließendes Erwärmen auf Raumtemperatur bewährt, während für die Herstellung der entsprechenden Dialkoxyverbindungen mit bis zu 5 C-Atomen je Alkylrest höhere Temperaturen vorteilhaft sind. Temperaturen von + 60° sollten jedoch nicht überschritten werden, da mit steigender Temperatur die Bildung unerwünschter Nebenprodukte begünstigt wird.

Bei der Durchführung des erfindungsgemäßen Verfahrens werden die Reaktionsteilnehmer in dem angegebenen Molverhältnis in Gegenwart von vorzugsweise trockenem Chlorwasserstoff zur Reaktion gebracht. Hierbei können entweder das 3-Chlor-1-hydroxyimino-aceton, gelöst in wasserfreiem Alkanol in eine Lösung aus wasserfreiem Alkanol, Chlorwasserstoff und Alkylnitrit oder das Alkylnitrit in eine Lösung aus 3-Chlor-1-hydroxyimino-aceton, wasserfreiem Alkanol und Chlorwasserstoff eingetragen werden, wobei jeweils durch kräftige mechanische Bewegung für eine rasche Verteilung der Reaktionspartner und durch ausreichende Kühlung für die Einhaltung der Reaktionstemperatur gesorgt wird. Nach beendeter Zugabe der Reaktionsteilnehmer wird die mechanische Bewegung des Reaktionsgemisches, gegebenenfalls unter Erwärmen fortgesetzt, bis die Stickoxidentwicklung beendet ist. Anschließend wird das überschüssige Alkanol unter schonenden Bedingungen, vorteilhaft unter vermindertem Druck abdestilliert, das so erhaltene Rohprodukt in einem inerten,polaren Lösungsmittel gelöst, durch Zugabe von festen Neutralisationsmitteln von restlichem Chlorwasserstoff befreit und dann durch Destillation unter vermindertem Druck in reiner Form isoliert. Unter Einhaltung dieser Bedingungen werden die 3-Chlor-1,1-dialkoxy-acetone mit einer Reinheit $>$ 90 % in guten Ausbeuten erhalten.

Als inerte polare Lösungsmittel sind solche mit Siedepunkten $<$ 100°C bevorzugt. Beispiele hierfür sind chlorierte aliphatische Kohlenwasserstoffe, wie Dichlormethan, Chloroform und Tetrachlorkohlenstoff, aliphatische Ether, wie Diethylether und Isopropylether und aliphatische Ester, wie Methylacetat und Ethylacetat. Als feste Neutralisationsmittel haben sich beispielsweise Calciumoxid und insbesondere Calciumcarbonat bewährt.

Die nach dem erfindungsgemäßen Verfahren herstellbaren 3-Chlor-1,1-dialkoxy-acetone sind schwach gelb gefärbte Flüssigkeiten, die bei Raumtemperatur stabil und längere Zeit ohne Zersetzungsneigung lagerbeständig sind.

Ihre Struktur wurde durch $^1$H-NMR-Analyse bestimmt.

Durch die folgenden Beispiele wird das erfindungsgemäße Verfahren näher erläutert.

Beispiel 1

Herstellung von 3-Chlor-1-hydroxyimino-aceton:

In einem 2 l-Dreihalskolben, der mit Rührer, Tropftrichter und Thermometer ausgerüstet war, wurde eine Lösung von 69 g Natriumnitrit in 800 ml Wasser vorgelegt, in die bei 0° bis +5°C eine Lösung von 136,5 g 4-Chloracetessigsäure in 680 g 5,26 %-iger Salzsäure innerhalb einer Stunde eingetragen wurde. Nach Erreichen der Raumtemperatur wurde das Reaktionsgemisch durch Zugabe von Natriumchlorid gesättigt und dreimal mit je 300 ml Diisopropylether extrahiert. Die vereinigten Etherphasen wurden zweimal mit je 50 ml einer gesättigten Natriumhydrogencarbonatlösung gewaschen und über Natriumsulfat getrocknet. Nach Entfernen des Lösungsmittels im Vakuum wurden 98,3 g 3-Chlor-1-hydroxyimino-aceton in Form von farblosen, blättchenartigen Kristallen erhalten. Die Ausbeute, bezogen auf 4-Chloracetessigsäure, betrug 81 % d.Th. Schmelzpunkt: 97° - 99°C.

Beispiel 2

a) In einen 1 l-Kolben, der mit Gaseinleitungsrohr, Thermometer, Rührer und Tropftrichter ausgerüstet war, wurden in 440 ml wasserfreies Methanol bei -5° 46 g gasförmiger Chlorwasserstoff eingeleitet. Dann wurden bei -20°C 41 g gasförmiges Methylnitrit eingeleitet. Bei -7° bis -10°C wurde anschließend eine Lösung von 72,9 g des nach Beispiel 1 hergestellten 3-Chlor-1-hydroxyimino-acetons in 100 ml wasserfreiem Methanol innerhalb von 45 Min. zugetropft.

Nach Erwärmen des Reaktionsgemisches wurde noch weitere 15 Minuten gerührt. Dann wurde das Methanol im Vakuum unter einem Druck von 2.660 Pa schonend abdestilliert. Der Rückstand wurde in 250 ml Methylenchlorid aufgenommen und mit 10 g $CaCO_3$ versetzt. Nach der Entfernung des Feststoffes wurde destilliert. Es wurden 67,4 g 3-Chlor-1,1-dimethoxy-aceton vom Siedepunkt 79° - 81°C/1333 Pa mit einer Reinheit von 92,4 % erhalten. Die Ausbeute betrug 68 % d.Th., bezogen auf eingesetztes 3-Chlor-1-hydroxyimino-aceton.

b) In 440 ml wasserfreies Methanol wurden bei -5°C 46 g gasförmiger Chlorwasserstoff eingeleitet. Dann wurden 72,9 g von nach dem in Beispiel 1 beschriebenen Verfahren hergestellten 3-Chlor-1-hydroxyimino-aceton, gelöst in 100 ml wasserfreiem Methanol eingetragen. In diese Lösung wurden unter Aufrechterhaltung einer Temperatur von -10° bis -7°C 41 g gasförmiges Methylnitrit eingeleitet. Nach Aufarbeitung des Reaktionsgemisches, wie unter a) beschrieben, wurden 62,8 g des 3-Chlor-1,1-dimethoxy-acetons mit einer Reinheit von 90,2 % erhalten. Die Ausbeute betrug 62 % d.Th., bezogen auf eingesetztes 3-Chlor-1-hydroxyimino-aceton.

Kenndaten des 200 MHz-$^1$H-NMR-Spektrums von 3-Chlor-1,1-dimethoxy-aceton

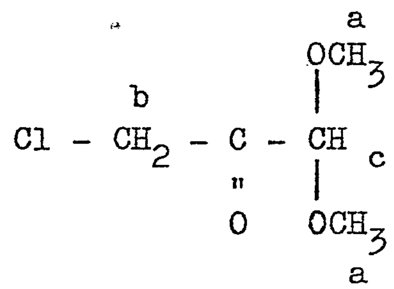

Lösungsmittel: $CDCl_3$

| Zuordnung der H-Atome | chemische Verschiebung $\delta$ [ppm*] | Multiplizität der Signale | relative Intensität der Signale |
|---|---|---|---|
| a | 3.3 | S | 6 |
| b | 4.3 | S | 2 |
| c | 4.5 | S | 1 |

*ppm, bezogen auf internes $CHCl_3$ bei 7,27 ppm

S = Singulett

<u>Beispiel 3</u>

In 400 ml wasserfreies Ethanol wurden bei -5°C 36,5 g gasförmiger Chlorwasserstoff eingeleitet. Nach der Zugabe von 65 g (0,54 Mol) 3-Chlor-1-hydroxyimino-aceton (hergestellt nach dem in Beispiel 1 beschriebenen Verfahren) wurden bei -5° bis -2°C 40,5 g (0,54 Mol) gasförmiges Ethylnitrit eingeleitet. Nach beendeter Gasentwicklung wurden 300 ml Wasser zugegeben und das Reaktionsgemisch 4 x mit je 300 ml Methylenchlorid extrahiert. Die vereinigten organischen Extrakte wurden durch Zugabe von 8 g $CaCO_3$ neutralisiert. Nach Entfernung des Lösungsmittels im Vakuum wurde destilliert. Es wurden 56,2 g (58 % d.Th.) 3-Chlor-1,1-diethoxyaceton mit Siedepunkt 78 - 80°C/700 Pa erhalten.

Kenndaten des 360 MHz-$^1$H-NMR-Spektrums von
3-Chlor-1,1-diethoxy-aceton

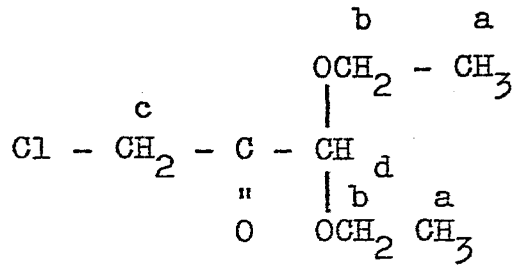

Lösungsmittel: $CDCl_3$

| Zuordnung der H-Atome | chemische Verschiebung $\delta$ [ppm*] | Multiplizität der Signale | relative Intensität der Signale |
|---|---|---|---|
| a | 1.1 | T | 6 |
| b | 3.6 | M | 4 |
| c | 4.3 | S | 2 |
| d | 4.6 | S | 1 |

*ppm, bezogen auf internes $CHCl_3$ bei 7,27 ppm

T = Triplett

M = Multiplett

S = Singulett

Beispiel 4

In 400 ml wasserfreies Isopropanol wurden bei -5°C 36,5 g gasförmiger Chlorwasserstoff eingeleitet. Nach der Zugabe von 65 g (0,54 Mol) 3-Chlor-1-hydroxyimino-aceton (hergestellt nach dem in Beispiel 1 beschriebenen Verfahren) wurden bei +5° bis +10°C 48,1 g (0,54 Mol) Isopropylnitrit zugetropft. Nach beendeter Gasentwicklung wurden 300 ml Wasser zugegeben und das Reaktionsgemisch 4 x mit je 300 ml Methylenchlorid extrahiert. Die vereinigten organischen Extrakte wurden durch Zugabe von 8 g festem $CaCO_3$ neutralisiert. Nach Entfernen des Lösungsmittels im Vakuum wurde destilliert. Es wurden 52,6 g (47 % d.Th.) 3-Chlor-1,1-dipropoxy-aceton mit Siedepunkt 76 - 78°C/500 Pa erhalten.

Kenndaten des 200 MHz-$^1$H-NMR-Spektrums von 3-Chlor-1,1-dipropoxy-aceton

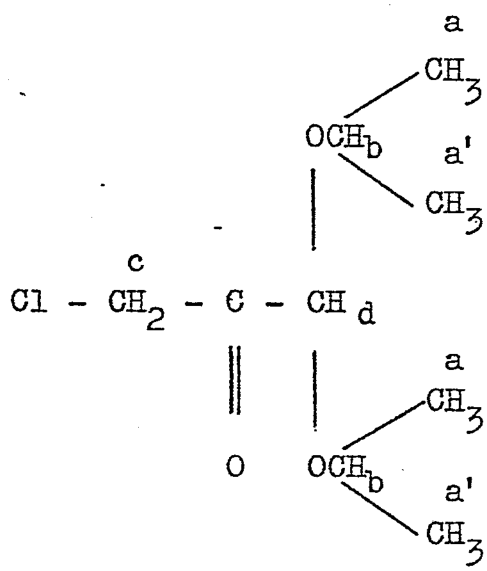

Lösungsmittel: $CDCl_3$

| Zuordnung der H-Atome | chemische Verschiebung δ [ppm*] | Multiplizität der Signale | relative Intensität der Signale |
|---|---|---|---|
| a | 1,07 | D | 6 |
| a' | 1,14 | D | 6 |
| b | 3,8 | M | 2 |
| c | 4,4 | S | 2 |
| d | 4,7 | S | 1 |

*ppm, bezogen auf internes $CHCl_3$ bei 7,27 ppm

D = Dublett          M = Multiplett          S = Singulett

Patentansprüche:

1. 3-Chlor-1,1-dialkoxy-acetone der Formel

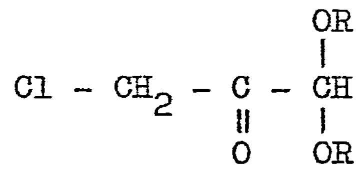

worin R Alkylreste mit 1 bis 5 C-Atomen bedeutet.

2. Verfahren zur Herstellung der 3-Chlor-1,1-dialkoxy-acetone,
d a d u r c h g e k e n n z e i c h n e t,
daß Gemische aus 3-Chlor-1-hydroxyimino-aceton und einem Alkanol mit Alkylnitriten in Gegenwart eines anorganischen Elektronenpaarakzeptors bei Temperaturen im Bereich von -25°C bis +60°C umgesetzt und die so erhaltenen Rohprodukte durch Destillation unter vermindertem Druck gereinigt werden.

3. Verfahren nach Anspruch 2,
d a d u r c h g e k e n n z e i c h n e t,
daß als Reaktionspartner 3-Chlor-1-hydroxyimino-aceton verwendet wird, das unmittelbar vor Gebrauch durch Umsetzung von 4-Chloracetessigsäure mit Natriumnitrit in wässeriger Lösung und anschließender Extraktion mit einem organischen Lösungsmittel erhalten worden ist.

4. Verfahren nach Anspruch 2,
d a d u r c h g e k e n n z e i c h n e t,
daß als Reaktionspartner Alkanole und Alkylnitrite mit jeweils identischen, 1 bis 5 C-Atome enthaltenden Alkylresten verwendet werden.

5. Verfahren nach Anspruch 2,
d a d u r c h g e k e n n z e i c h n e t,
daß als anorganischer Elektronenpaarakzeptor Chlorwasserstoff verwendet wird.

6. Verfahren nach Anspruch 2 und 4,
d a d u r c h g e k e n n z e i c h n e t,
daß je Mol des 3-Chlor-1-hydroxyimino-acetons 5 bis 20 Mole des Alkanols und 1,0 bis 1,2 Mol des Alkylnitrits verwendet werden.

7. Verfahren nach Anspruch 2 und 5,
d a d u r c h g e k e n n z e i c h n e t,
daß je Mol des 3-Chlor-1-hydroxyimino-acetons 0,01 bis 2 Mol Chlorwasserstoff verwendet werden.

8. Verbindung gemäß Anspruch 1, die durch Umsetzung von 3-Chlor-1-hydroxyimino-aceton und Methanol mit Methylnitrit in Gegenwart von Chlorwasserstoff hergestellt worden ist, mit einem Siedepunkt von 79° bis 81°C/1333 Pa.

Europäisches Patentamt

# EUROPÄISCHER RECHERCHENBERICHT

0122479

Nummer der Anmeldung

EP 84 10 2923

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| X | CHEMISCHE BERICHTE, Band 99, Nr. 12, 1966, Weinheim-Bergstrasse H. BAGANZ et al. "Neue Synthesen von Hydroxymalondialdehyd(Triosereduk ton)", Seiten 3771-3777 * Seite 3773, Formel 13 * --- | 1 | C 07 C 49/175<br>C 07 C 45/00 |
| X | DE-B-1 254 138 (NORDMARK-WERKE) * Beispiel 2; Spalte 5, Zeile 4 * --- | 1 | |
| A,D | US-A-4 158 019 (K. MEYER) * Spalte 1, Zeilen 47-59 * ----- | 2 | |

RECHERCHIERTE SACHGEBIETE (Int. Cl. 3)

C 07 C 45/00
C 07 C 45/64
C 07 C 45/65
C 07 C 45/68
C 07 C 49/175

Der vorliegende Recherchenbericht wurde für alle Patentansprüche erstellt.

| Recherchenort | Abschlußdatum der Recherche | Prüfer |
|---|---|---|
| BERLIN | 04-06-1984 | KNAACK M |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03.82